# EUROPEAN PATENT SPECIFICATION

(11) **EP 2 700 318 B1**
(45) Date of publication and mention of the grant of the patent: **21.08.2019**
(21) Application number: 12777065.9
(22) Date of filing: 28.03.2012
(51) Int. Cl.: A01N 63/04

(54) **USES, METHODS AND BIOLOGICAL COMPOSITIONS OF THE GENUS PAECILOMYCES FOR THE CONTROL, PREVENTION AND ERADICATION OF PHYTOPARASITES IN SOLANACEAE CULTURES**
VERWENDUNGEN, VERFAHREN UND BIOLOGISCHE ZUSAMMENSETZUNGEN DER GATTUNG PAECILOMYCES ZUR BEKÄMPFUNG, PRÄVENTION UND ELIMINIERUNG VON PFLANZENPARASITEN IN SOLANACEAE-KULTUREN
UTILISATIONS, MÉTHODES ET COMPOSITIONS BIOLOGIQUES DU GENRE PAECILOMYCES POUR LA RÉGULATION, LA PRÉVENTION ET D'ÉRADICATION DE PHYTOPARASITES DANS DES CULTURES DE SOLANACÉES

(30) Priority: 29.04.2011 MX MX11004510
(43) Date of publication of application: 26.02.2014
(73) Proprietor: Instituto De Ecología, A.C., C.P. 91070 Xalapa, Veracruz (MX)
(72) Inventor: CARRION VILLARNOVO, Gloria Luz Laura, CP. 91 150 Xalapa, Veracruz (MX); HERNANDEZ LEAL, Tania Isadora, CP. 91010 Xalapa, Veracruz (MX); LOPEZ LIMA, José Daniel, CP. 91098 Xalapa, Veracruz (MX); NUÑEZ SANCHEZ, Angel Enríque, CP. 91020 Xalapa, Veracruz (MX)
(74) Representative: Stiebe, Lars Magnus
(86) International application number: PCT/MX2012/000032
(87) International publication number: WO 2012/148251

(56) References cited:
- ANGEL ENRIQUE NUNEZ SANCHEZ: "Aislamiento y evaluación de hongos nematófagos asociados a quistes de Globodera rostochiensis (Woll.) en la región del Cofre de Perote", INTERNET CITATION, 1 January 2002 (2002-01-01), page 107pp, XP008171369, Retrieved from the Internet: URL:http://digeset.ucol.mx/tesis_posgrado/ Pdf/Angel%20Enrique%20Nu%C3%B1ez%20Sanchez .pdf [retrieved on 2014-08-20]
- LOPEZ-LLORCA L V ET AL: "NEW MEDIA FOR THE ESTIMATION OF FUNGAL INFECTION IN EGGS OF THE CEREAL CYST NEMATODE HETERODERA-AVENAE WOLL", NEMATOLOGICA, BRIL, LEIDEN, NL, vol. 32, no. 4, 1 January 1986 (1986-01-01), pages 486-489, XP009179911, ISSN: 0028-2596
- NÚÑEZ, A. E.: 'Aislamiento y evaluación de hongos nematófagos asociados a quistes de Globodera rostochiensis (Woll.) en la región del Cofre de Perote.' TESIS FACULTAD DE CIENCIAS BIOLÓGICAS Y AGROPECUARIAS. 2002, COLIMA, page 1, 50, 66, 75, 78 AND 93, XP008171369
- ORTEGA, E.: 'El nematodo quiste de la papa. II: Gestión integrada de control y prospectiva de la restricción cuarentenaria para la producción y distribución de tubérculo-semilla en el Mundo.' REVISTA DIGITAL INIA HOY January 2008, XP055169151 Retrieved from the Internet: <URL:http://www..inia.gov.ve/index.php?opti on=com-content&task=view&id=460 [retrieved on 10/10/12>

## Description

### FIELD OF THE INVENTION

This invention relates to the use of fungus Paecilomyces carneus in the control or prevention or eradication of Globodera spp. in cultures parasited by cyst-forming nematodes, implemented simultaneously with a culture rotation, and wherein the fungus Paecilomyces carneus is mass propagated in a composition comprising oat, yeast, carrot juice and chloramphenicol.

This invention has the advantage of being innocuous both for the product, the soil, and the producer and end consumer, as it does not contribute in the obtainment of an organic product.

### BACKGROUND OF THE INVENTION

Nematodes are generally multi-cellular filiform microscopic plant parasites, with reduced diameter ends. Generally, nematodes are dioic (there are males and females) and the reproduction thereof implies a crossed fecundation. As per their biologic cycle this has six stages: a cyst, four juvenile stages (J1, J2, J3 and J4) and adult. Cysts remain into the soil infecting the roots of cultures during the following culture cycle. A nematode may have up to six generations during a culture time depending on the date of seeding, the temperature of the soil, the length of the growing time, the type of host and the geographic zone. Cysts can remain viable for long periods of time of up to twenty years.

Generally, nematodes, upon infecting cultures, produce aerial damages, such as a reduction in the seeds germination, low production, foliar stains, decay and chlorosity, dwarfism, leaf and stem deformation, internal necrosis in stem, defoliation and lack of greenness and under earth damages such as nodulated roots, detaching and necrosis of the skin on roots, corms and tubercles, low production of seeds and buds or shoots, deformation and necrosis of roots, bulbs, tubercles and corns, excess proliferation of roots, atrophy of radical apexes or an underdeveloped radical system.

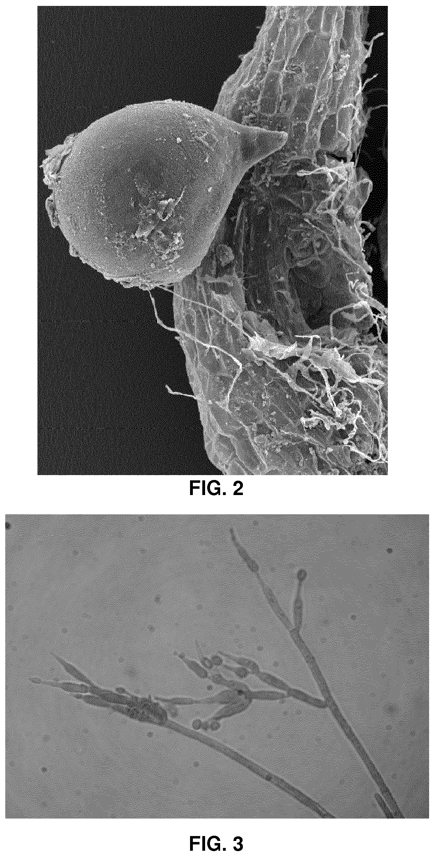

An infestation by *Globodera rostochiensis* (Figure 2) is produced when larvae J2 burst under the stimuli from radicals exuded out from the plant. Said J2 enter the apical portion of the root, into the epidermal cells and the cellular walls through the stylus thereof, and start feeding from the pericycle cells. Thus, the infested plants become subject to a reduced roots system, rachitis, dwarfism and chlorosis; and due to a reduction in the water absorption the plant may die.

Golden nematode or *Globodera rostochiensis* is a cyst-forming nematode infecting food and ornamental plants, with million dollars loses in a year. This nematode has affected potato-producing zones back from years 70s and from then it has spread to lukewarm and/or mountain zones where said tubercle is grown. Under a conservative appreciation, this nematode produces In Mexico loses for about 800,000 tons of potato production yearly, equivalent to about 3000 million Mexican pesos yearly. In Veracruz state the loses are esteemed in about 50,000 tons of potatoes yearly, equivalent to about 415 million Mexican pesos yearly, since a statistical lowering in the potato production has been observed in Veracruz state (OEIDRUS, 2008).

During 2008 a production of 300 million tons of potato was reported in the world over, with the Republic of China being the main provider and consumer thereof. This food shows a high nutritional value, is a versatile and noble food, has a variety of uses of industrial character, such as starch, maltose, dextrose, molasses and glucose.

According to United Nations' Food and Agriculture Organization (FAO), the world production of potato in 2007 was 325.30 million tons, the main producers being China, Russian Federation, India, United States, Ukraine, Poland, Belarus, Netherlands and France. On the other side, the main consumers of this tubercle are European countries whilst the lower consumers are in Africa and Latin America.

According to the Secretary of Agriculture, Cattle, Rural Development, Fishing and Food (SAGARPA) potato is ranked in Mexico in the 35th place for seeded surface; however, regarding the production value, this tubercle is ranked in the 9th place. More than 8700 producers are dedicated to potato farming in 12 states of the country, the farming of which generates 17,500 direct employments, 51,600 indirect ones and 6.3 million journals every year (2008).

In Veracruz state the mountain zones, such as the slopes of Cofre de Perote and Pico de Orizaba, are the ideal places for growing potatoes. Since 1987 the entire producing zone (6000 Ha) of Veracruz state has been under a quarantine according to the official document "Permanent Interior Quarantine Number 17 against the Potato Golden Nematode *Globodera rostochiensis",* as published in 1987 and in force nowadays, through the Mexican Official Standard NOM040-FITO-2002, wherein requirements and specifications are given for the production and national mobilization of commercial potato, forbidding the use of said tubercle for seeds since this can be a propagator vehicle of nematodes, specifically of *Globodera rostochiensis.* This forbidding has been disrespected thus resulting in a greater dispersion of this noxious plague. These rules show that the problem has remained unsolved for more than 30 agricultural cycles.

Basically, the golden nematode or *Globodera rostochiensis* has affected the Mexican potato-producing zones as back as from about 1972; since it was then when it scattered to the mountain zones where this tubercle is grown. The records of the presence of said parasite show that it reached populations 100 times greater than the recommended value in order to avoid the damage to the production (i. e., 40 cysts per soil kg), according to the European Organization for Plant Protection.

The most commonly employed methods for controlling *Globodera rostochiensis* are the application of nematicides, such as carbofuran, which, further of being toxic, is not a definitive solution. There are in the state of the art methods for controlling nematodes of the *Globodera spp.* by means of the use of synthetic proteins (WO 01/94601 A2), the use of 2R,5R-dihydroxymethyl-3R, R-dihydroxypirrolidine (WO/1999/05941; a chemical agent obtained from plants of the Solanaceae family (WO/1999/059414); through the use of proteinase inhibitors (WO/1992/015690); and by the use of Bacillus thuringiensis (WO/1993/019604); compositions with one or more essential oils from *Tagetes erecta, Ocimum basilicium* or *Cymbopongon martini*; a carrier and an emulsifier (WO/2007/13222); compositions based on the bark of Afrostyrax lepidephyllus (WO/1995/010187), addition of cisterna proteinase (WO/2008/087555) and, specifically for the control of *Globodera rostochiensis,* the use of *Arthrobotrys sp.* (KR20030094540), which is too much sensitive to ambient factors and is inoperable on de-structured soils; chemical agents obtained from Solanaceae (JP3190807); and a chemical agent of the condensed formula C₂₇H₃₀O₉ (WO/1993/002083). These control methods, jointly with the strategy of culture rotation, are generally carried out in a disorganized manner and with scarce knowledge on the nematode cycle of life. This and the doses employed, cause pollution and damages to the freatical mantles, pollution of the product and, in some instances, generate resistance on said nematodes and, consequently, making the eradication thereof more difficult.

The genus *Paecilomyces spp.* has being used in the following applications: *Paecilomyces tenuipes* in the control of flying insects in plants (Patents No. No. US 7,033,586 B2, US 20030124098 and US 2007 0141032); a composition containing *Paecilomyces lilacinus* used in the control of soil pests (US 7,435,411 B2, CN101418264 and US 20050008619); *Paecilomyces fumosoroseus or Paecilomyces javanicus* in the control of under earth thermites (US 7,390,480); *Paecilomyces javanicus,* combined with imidacloprid in the control of sucker insects (CN101502270); *Paecilomyces javanicus* combined with azadirachtin (CN101331880 and CN101331884); *Paecilomyces fumosoroseus* combined with azadirachtin (CN101273728 and WO/1991/011856); a protein obtained from *Paecilomyces farinosus* as an insecticide (WO/2001/000841); and more particularly, in the control of plants parasite nematodes among which there are: *Paecilomyces lilacinus* (Pat. No. US 5,989,54, Pat No. CN101422168, Pat No. DE102005024783, Pat No. CN101081982 and Pat No. CA2059642); *Paecilomyces fumosoroseus* (US 5,360,607); *Paecilomyces lilacinus* 251, *Paecilomyces lilacinus* 252, *Paecilomyces lilacinus* 253 and *Paecilomyces lilacinus* 254 (US 5,989,543); and *Paecilomyces cicadae* (CN101518265), without any mention to the use of *Paecilomyces carneus* in the control of nematodes in crops. ANGEL ENRIQUE NUNEZ SANCHEZ: "Aislamiento y evaluacion de hongos nematofagos asociados a quistes de Globodera rostochiensis (Woll.) en la region del Cofre de Perote", URL:http://digeset.ucol.mx/ tesis_posgrado/Pdf/Angel%20Enrique%20Nu%C3%Blez%20Sanchez.pdf, provides a clear incitation for the skilled artisan to consider the control of Globodera nematodes by means of Paecilomyces fungi.

According to the above discussed, and as a response to a problem of such an important market throughout the world as the potato market, this invention has been developed to provide an optimized method of production of *Paecilomyces carneus,* as well as uses, methods and compositions easy to be applied in the control and/or prevention and/or eradication of nematodes in Solanaceae, and without a negative effect neither on the soil and on the product or the final consumer.

### OBJECTS OF THE INVENTION

The present invention relates to the use of fungus *Paecilomyces carneus,* singly or combined with at least one carrier, in the control or prevention or eradication of *Globodera* spp. in cultures parasited by cyst-forming nematodes, implemented simultaneously with a culture rotation, and wherein the fungus *Paecilomyces carneus* is mass propagated in a composition comprising oat, yeast, carrot juice and chloramphenicol.

### BRIEF DESCRIPTION OF THE DRAWINGS

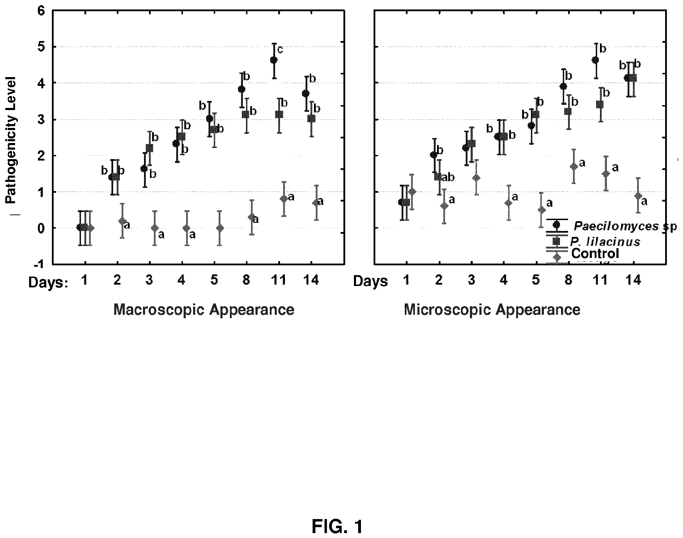

FIGURE 1. A comparative study of the effect of Paecilomyces carneus and *Paecilomyces lilacinus* on *Globodera rostochiensis.*
FIGURE 2. A microphotography of strain IE-431 of *Paecilomyces carneus.*
FIGURE 3. A microphotography of *Globodera rostochiensis.*
FIGURE 4. *Globodera rostochiensis* cysts.
FIGURE 5. J2 of *Globodera rostochiensis* infected with *Paecilomyces carneus* strain IE-431, inside the egg.

### DETAILED DESCRIPTION OF THE INVENTION

This invention disclosed herein below comprises the use of *Paecilomyces carneus* for the control and/or prevention and/or eradication of plant parasites in Solanaceae cultures.

*Paecilomyces carneus* strain IE-431 is a fungus produced according to the methods of this invention. Said strain is presently maintained and registered in the fungal collection of the Instituto de Ecologia (INECOL), and is registered with the World Federation Culture Collection (WFCC) under No. IEWDCM782. The isolation and/or maintain of the Paecilomyces carneus strain IE-431 are carried out in one or more solid culture means, constituted by at least a nitrogen source and/or at least a carbon source and/or one or more mineral salts and/or at least a suitable vehicle and/or at least an antibiotic agent and/or at least a growing helper agent. Said culture is incubated within a temperature within the range of from 12 °C to 35 °C for a time enough to obtain a pertinent concentration.

The massive reproduction of the fungus consists in the inoculation of living cells of *Paecilomyces carneus* in liquid media comprising one or more amino acids and/or one or more carbohydrates and/or one or more mineral salts and/or at least one suitable vehicle and/or at least an antibiotic agent and/or at least a growing helper agent, however at least oat, yeast, carrot juice and chloramphenicol. Said culture is incubated at a temperature within a range of from 12 °C to 35 °C for a time enough to obtain a pertinent concentration (FIGURE 3).

Said fungus *Paecilomyces carneus* strain IE-431 - obtained from a process of isolation and/or maintain and/or massive reproduction disclosed in this investigation-, exhibits a series of genetic characteristics that are conserved without representative structural changes, which make it different from any other species of Paecilomyces and never observed before with any other species belonging to genus *Paecilomyces spp.,* characterized in that presents a colony diameter of from 15 to 21 mm in 10 days on agar-oats (AA) media, of from 15 to 19 mm on Agar-dextrose-potato (PDA), while on Agar-carrot-potato (PZA) it grows from 15 to 17 mm. The mycelium thereof is of white to hyaline color (1A1) in the color chart of Farver and Faver of Wanscher and Kornerup (1961); it does not stain the culture media tested both on the front and reverse of the agar plate. The texture thereof is filamentous accented on PZA; it grows very close to the surface of the media (sparse) and becomes dusty when sporulates. Said mycelium deforms the PDA with the appearance of notorious grooves at the reverse of the box. It is shaped as erect, macronematous, simple conidiophores, with no branches with catenulated equinulated conidia of subglobe shape, and some of them have ends slightly pointed at the ends, dimensioned to 2.4-4 µm x 2-3.2 pm.

In the solid culture medium, 86% of the conidia germinate at 16 hours, the germ tube varies between 6 and 54 micrometers in length. At 24 hrs 100% of the spores germinate and the mycelium is greater than 30 and less than 110 micrometers in length.

Under the light of the above discussed technical knowledge developed for this invention, we are filing the DNA nucleotide sequence coding *Paecilomyces carneus* strain IE-431, as disclosed at the end hereof.

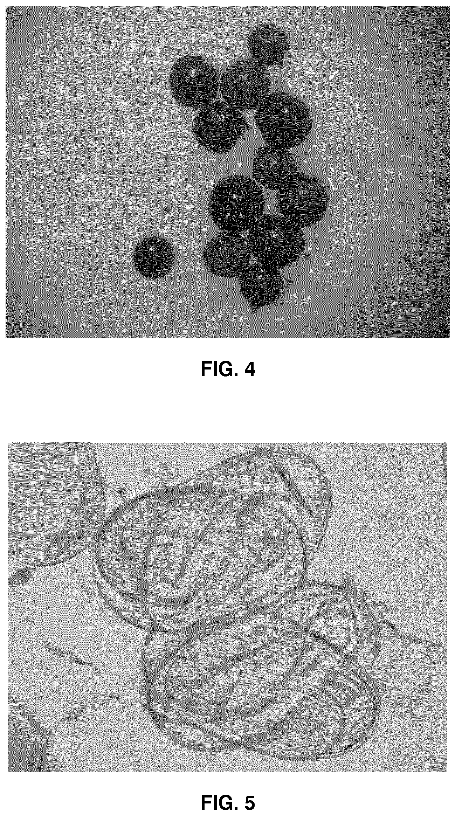

In this investigation, it was surprisingly found that, when living cells of *Paecilomyces carneus* IE-431 were placed in contact with cysts of the nematode *Globodera rostochiensis,* substantial damages were produced on the cysts, such that the propagation of this parasite is avoided. During our investigations, we found that the interaction between *Paecilomyces carneus* and *Globodera rostochiensis* occurs through the fungus germination which, when contacts the cysts (figure 4) said cysts are colonized by the mycelium at the interior thereof (figure 5) thus surprisingly affecting the whole amount of eggs and larvae J1 and J2 contained within said cyst. The cyst infection starts through an enzymatic process when *Paecilomyces carneus* permeates the cyst wall and then the mycelium grows on the surface of the eggs and the interior of juvenile J1 and J2; therefore, the development of said mycelium becomes invasive to the point of completely degrade the eggs and/or juveniles J1 and J2.

During the investigation a study was carried out on the nematicide effect of *Paecilomyces carneus* strain IE-431 against the effect of *Paecilomyces lilacinus* IE-430 on the *Globodera rostochiensis* cysts, and It was surprisingly found that *Paecilomyces carneus* IE-431 acts in a more rapid and noxious manner against the cysts of said nematode; i. e., there is a significant difference between to pathogenicity of one species and the other against said nematode. This study was carried to laboratory level, evaluating the degree of infection of said cysts with a concentration of living cells of either fungus of 1 x 10⁶ living cells/mL, and a third control group with the only addition of distilled sterile water. Said pathogenicity was evaluated for a period of 14 days, according to the macroscopic and microscopic aspects of cysts, and a significant unexpected difference was obtained relating the effect of fungus *Paecilomyces carneus* strain IE-431, compared to *Paecilomyces lilacinus.* Results are shown in Figure 5.

The next test stage of the use of fungus *Paecilomyces carneus* strain IE-431 as a nematicide was applied singly (TP) and jointly with lima bean (HP) and peas (ChP) crops rotation, with an absolute witness (TA) where neither control nor crop were applied. Said lima bean and peas crops with no treatment with said fungus, are considered as witnesses (H) and (Ch). When the population density is above 200 cysts/100 g of soil (viability average 10-40 and up to 100 eggs/g soil) the first application is effected before seeding; a second application is made during the seeding and a third application is carried out after the harvest of lima bean and peas in order to provide for a decreasing in the golden nematode population down to practically zero (Table 1 and Table 2).

**Table 1. Effect of Paecilomyces carneus on the control of the potato golden nematode, compared to the lima bean crop in rotation and non-farmed plots of land.**

| parameters | Monitoring | Treatments | | | |
|---|---|---|---|---|---|
| | | H | HP | TP | TA |
| Cysts 100 g | 1 | 227 | 272 | 171 | 180 |
| | 2 | 155 | 144 | 200 | 189 |
| | 3 | 140 | 102 | 154 | 183 |
| | 4 | 133 | 101 | 153 | 211 |
| Viability 1 g | 1 | 40 | 64 | 13 | 15 |
| | 2 | 14 | 14 | 20 | 13 |
| | 3 | 12 | 7 | 11 | 14 |
| | 4 | 15 | 5 | 9 | 15 |
| J2-Gr 100 g | 1 | 4 | 14 | 3 | 4 |
| | 2 | 7 | 9 | 4 | 3 |
| | 3 | 21 | 6 | 12 | 16 |
| | 4 | 17 | 2 | 3 | 20 |
| Free-living 100 g | 1 | 62 | 131 | 119 | 124 |
| | 2 | 148 | 137 | 78 | 70 |
| | 3 | 72 | 82 | 61 | 52 |
| | 4 | 42 | 72 | 57 | 43 |

**Table 2. Effect of Paecilomyces carneus on the control of the potato golden nematode, compared to the peas crop in rotation and non-farmed plots of land.**

| parameters | Monitoring | Treatments | | | |
|---|---|---|---|---|---|
| | | cH | chP | TP | TAC |
| Cysts 100 g | 1 | 151 | 173 | 250 | 237 |
| | 2 | 139 | 134 | 139 | 109 |
| | 3 | 146 | 95 | 126 | 130 |
| | 4 | 128 | 118 | 124 | 99 |
| Viability 1 g | 1 | 24 | 19 | 17 | 26 |
| | 2 | 17 | 19 | 15 | 19 |
| | 3 | 15 | 13 | 14 | 15 |
| | 4 | 8 | 7 | 9 | 10 |
| J2-Gr 100 g | 1 | 5 | 3 | 11 | 10 |
| | 2 | 1 | 3 | 11 | 8 |
| | 3 | 9.8 | 13 | 8 | 18 |
| | 4 | 6 | 8 | 4 | 16 |
| Free-living 100 g | 1 | 73 | 105 | 84 | 70 |
| | 2 | 73 | 101 | 80 | 69 |
| | 3 | 61 | 55 | 69 | 72 |
| | 4 | 62 | 56 | 115 | 50 |

As can be seen from the above, *Paecilomyces carneus* strain IE-341 has a nematicide effect on the viability of the potato golden nematode cysts but no action on free life nematodes, which are to be kept on farming soils. Under this investigation, we found a synergic behavior for reducing the population density of golden nematode when the application of *Paecilomyces carneus* is combined with the rotation of cultures that are not favorable to the bursting of J2 *Globodera rostochiensis.*

It is to be said that the use of rotation with lima bean and peas without the application of *Paecilomyces carneus* as a bionematicide is also effective, but requires of a period of time greater than 4 years.

Following an exhaustive investigation, a method is provided for isolation, maintaining, massive reproduction of Paecilomyces carneus strain IE-431, as well as the use thereof in the control and/or prevention and/or eradication of cyst-forming nematodes *Globodera rostochiensis* on cultures of the Solanaceae family, such as potato, chili, tomato, eggplant, among other plants where this type of parasite grows, such as *Avena sativa* and *Vicia villosa* (Winter Veza), and tobacco.

The composition preferred for application to farmings is in a suspension shape; however, these compositions can be developed and applied in the shape of pellets, powder, capsules, emulsion, microemulsion, solution or any other form of application existing in the state of the art.

### EXAMPLES

Herein below, intended to be descriptive are provided compositions employed for the propagation of fungus *Paecilomyces carneus* strain IE-431 and the nematicide compositions obtained.

### Example (comparative) 1: Composition A for mass propagation of Paecilomyces carneus strain IE-431.

| INGREDIENT | AMOUNT |
|---|---|
| Carrot juice | 80-100 mL |
| Ampicilina | 500 mg |
| Water | q.s. 1000 mL |

### Example 2: Composition B for mass propagation of Paecilomyces carneus strain EI-431.

| INGREDIENT | AMOUNT |
|---|---|
| Oats | 20-50 gL |
| yeast | 0.1-5.0 g/ l |
| Carrot juice | 50-150mL7L |
| Chloramphenicol | 1000 mg |
| Water | q.s. 1000mL |

### Example (comparative) 3: Composition C for isolation and maintain of Paecilomyces carneus strain IE-431.

| INGREDIENT | AMOUNT |
|---|---|
| Carrot | 50-160 g/L |
| Oats | 20-50 g/L |
| Chloramphenicol | 1000 mg |
| Water | c.b.p. 1000mL |

### Example (comparative) 4: Nematicide composition A-1

| INGREDIENT | AMOUNT |
|---|---|
| Living cells of *P. carneus* | 0.5 x 10⁷/mL |
| Composition A | 1000 mL |

### Example 5: Nematicide composition B-1

| INGREDIENT | AMOUNT |
|---|---|
| Living cells of *P. carneus* | 3 x 10⁷/mL |
| Composition B | 1000 mL |

### Example (comparative) 6: Nematicide composition C-1

| INGREDIENT | AMOUNT |
|---|---|
| Living cells of *P. carneus* | 0.3 x 10⁷/mL |
| Composition C | 1000 mL |

In an integral manner, this invention provides the following advantages:
1.- A method of agricultural control, based on clean and supporting technologies.
2.- Said method produces an improvement on the soil quality since allows for the recovery of soils used in Solanaceae cultures.
3.- The time to eradicate nematodes is one cycle, whereby it may stop the permanent interior quarantine number 17 against the potato golden nematode *Globodera rostochiensis.*
4.- An immediate improvement is produced on the quality of the obtained product, enlarging the shelf life thereof.
5.- The nematicide compositions obtained are innocuous for both humans and animals.
6.- The population of the golden nematode is reduced from the very first application.
7.- The cysts are directly attacked whereby better agricultural products are obtained from the very first application.

### Organization Applicant

Street: CARRETERA ANTIGUA A COATEPEC No. 351 COLONIA EL HAYA
City : XALAPA
State: VERACRUZ
Country: MEXICO
Postal Code: 91070
Phone number: (228)8421801
<110> Organization name: INSTITUTO DE ECOLOGÍA A.C.

### Individual Applicant

Street: CALLE PICACHO NO. 22, FRACCIONAMIENTO EL PEÑASCAL,
City: XALAPA
State: VERACRUZ
Country: MEXICO
Postal Code: 91150
Phone number: (228)8421800
<110> Last name: CARRION VILLARNOVO
<110> First name: GLORIA LUZ LAURA

### Aplication project

<120> Title: USOS, MÉTOPDOS Y COMPOSICIONES BIOLÓGICAS DEL GÉNERO PAECILOMYCES PARA EL ONTROL, PREVENCIÓN Y ERRADICACION DE FITOPARÁSITO EN CULTIVOS DE SOLANÁCEAS
<130> AppFileReference: World Federation culture collection (WFCC)No. IEWDCM782
<140> currentAppNumber:
<141> Current Filing Date:

### Sequence

<213> Organism Name: Paeci lomyces carneus cepa IE-431
<400> PreSequenceString: 
tccgtaggtg aacctgcgga gggatcatca ccgagttnac aactcccaaa ccccctgtga 60

acttatacca tttactgttg cttcggcggg tcacggcccc ggggaaggac agcggtcgcc 120

gtcaggcctc agctgcccgc ccccggaaca ggcgcccgcc ggggactcaa ctcttctgta 180

tttctttatc taatatatac tgtctgagta aaaactaaaa tgaatcaaaa ctttcaacaa 240

cggatctctt ggttctggca tcgatgaaga acgcagcgaa atgcgataag taatgtgaat 300

tgcagaattc agtgaatcat cgaatctttg aacgcacatt gcgcccgcca gtattctggc 360

gggcatgcct gttcgagcgt catttcaacc ctcaagtccc ctgtggactc ggtgttgggg 420

accggcgaga cagccgcgga tcttcttccg cagcgagtcg ccgcccccca aatgacttgg 480

cggcctcgtc gcggccctcc tctgcgtagt atagcacacc tcgcaacagg agcccggcga 540

atggcactgc cgtaaaaccc cccaactttt tcagagttga cctcgaatca cgtaggaata 600

cccgctgaac ttaagcatat ca 622
<212> Type: RNA
<211> Length 622

SequenceName: internal transcribed spacer 1, 5.8 ribosomal RNA gene sequenceDescription:

### Organization Applicant

Street: CARRETERA ANTIGUA A COATEPEC No. 351 COLONIA EL HAYA,
City: XALAPA
State: VERACRUZ
Country: MEXICO
Postalcode : 91070
Phone number: (228)8421801
<110> Organization name: INSTUTUTO DE ECOLOGÍA A.C.

### Individual applicant

Street: CALLE PICACHO No. 22, FRACCIONAMIENTO EL PEÑASCAL,
City: XALAPA
State: VERACRUZ
Country: MEXICO
Postal code: 91150
Phone number : (228)8421800
<110> Last name: CARRION VILLARNOVO
<110> First name: GLORIA LUZ LAURA

### Application Project

<120> Title: USOS, MÉTODOS Y COMPOSICIONES BIOLÓGICAS DEL GÉNERO PAECILOMYCES PARA EL CONTROL, PREVENCIÓN Y ERRADICACIÓN DE FITOPARÁSITOS EN CULTIVOS DE SOLANACEAS
<130> AppFileReference: World Federation Culture Collection (WFCC)NO.
   IEWDCM782
<140> CurrentAppNumber:
<141> CurrentFilingDate:

### Sequence

<213> Organism name: Paeci 1 omyces carneus cepa IE-431
<400> PreSequencestring: 
tcgattagtc tttcgccccc atgcccatat ttgacgatcg atttgcacgt cagaaccgct 60

gcgagcctcc accagagttt cctctggctt caccctatac aggcatagtt: caccttcttt 120

cgggtccggc cccgtatgct cttactcaaa tccatccgag aacatcagga tcggtcgatg 180

atgcgccgaa gctctcacct gcgttcactt tcattacgcg taggggtttt acacccgaac 240

acttgcatac gaagacgact ccttggtccg tgtttcaaga cgggtcactg atgaccatta 300

cgccagcatc cttgcacgaa tgcgcgaacc tcagtccgcc ccagggtatt gcgcagtggg 360

ctataacact cccggaggag ccacattccc agagcctttg tcccccggag cgaactgatg 420

ctggcctgag cccggcaaag tgcaccagcg acgagcgctg gatgattcac cgggcccaag 480

tctggtcatg agcgcttccc tttcaacaat ttcacgtnct atttaaccct cttttcaaag 540

tgcttttcat ctttcgatca ctctacttgt gcgctatcgg tctctggcca atatttagct 600

ttagaagaca tatacctccc atttagagca gcattcccaa actactcgac tcgtcgaagg 660

agctttacag aggctcggta l:ccaaccaga cggggctctc accctctatg gcgtcccgtt 720

ccagggaact cggaaggcac cttgccaaaa gcatcctctg caaannacaa ctcggaccct 780

gggggccaga tttcaaattt gnnnnnntgc cgctcactcg ccgttactgg ggcaat 836
<212> Type: RNA
<211> Length: 836
sequenceName: Paeci 1 omyces carneus cepa IE-431 large subunit ribosomal RNA gene partial sequence

SequenceDescription:

## Claims

1. Use of fungus *Paecilomyces carneus,* singly or combined with at least one carrier, in the control or prevention or eradication of Globodera spp. in cultures parasited by cyst-forming nematodes, implemented simultaneously with a culture rotation, and wherein the fungus *Paecilomyces carneus* is mass propagated in a composition comprising oat, yeast, carrot juice and chloramphenicol.

2. The use according to claim 1, wherein the culture parasited by cyst forming nematodes is a Solanaceae culture.

3. The use according to claims 1 to 2, singly or combined with at least one carrier, for the control or prevention or eradication of Globodera spp., wherein at least one application is carried out prior to the culture and/or during the culture and/or after every culture cycle.

4. A method for the control or prevention or eradication of Globodera spp. in cultures parasited by cyst-forming nematodes, said method comprising:
a. inoculating living cells of *Paecilomyces carneus* in a liquid media comprising oat, yeast, carrot juice and chloramphenicol;
b. incubating the culture at a temperature between 12°C and 35°C for sufficient time to obtain the desired concentration;
c. applying the *Paecilomyces carneus* living cells thus obtained, singly or combined with at least one carrier to cultures parasited by cyst-forming nematodes, simultaneously with a culture rotation.

5. The method according to claim 4, wherein the culture parasited by cyst forming nematodes is a Solanaceae culture.

6. The method according to claims 4 to 5, wherein at least one application is carried out prior to the culture and/or during the culture and/or after every culture cycle.

## Patentansprüche

1. Einsatz von *Paecilomyces carneus,* alleine oder in Verbindung mit mindestens einem Träger, zur Kontrolle oder Prävention oder Eradikation von *Globodera* spp. in Kulturen die von zystenbildenden Nematoden befallen sind, mit gleichzeitiger Durchführung von Kulturfolgen, in denen *Paecilomyces carneus* massenhaft in einer Zusammensetzung aus Hafer, Hefe, Karottensaft und Chloramphenicol propagiert wird.

2. Einsatz laut Anspruch 1, worin die von zystenbildenden Nematoden befallene Kultur eine Solanaceae Kultur ist.

3. Einsatz laut Anspruch 1 und 2, alleine oder in Verbindung mit mindestens einem Träger, zur Kontrolle oder Prävention oder Eradikation von *Globodera* spp. Mindestens eine Anwendung findet vor der Kultur und/oder während der Kultur und/oder nach jedem Kulturzyklus statt.

4. Eine Methode zur Kontrolle oder Prävention oder Eradikation von *Globodera* spp. in Kulturen die von zystenbildenden Nematoden befallen sind. Diese Methode beinhaltet:
a. Beimpfung lebender *Paecilomyces carneus* Zellen in einem flüssigen Medium aus Hafer, Hefe, Karottensaft und Chloramphenicol;
b. Inkubation del Kultur zwischen 12°C und 35°C bis die gewünschte Konzentrierung erreicht wird;
c. Aufbringen der so erhaltenen lebenden Zellen von *Paecilomyces carneus* , alleine oder in Verbindung mit mindestens einem Träger auf Kulturen, die von zystenbildenden Nematoden befallen sind und gleichzeitige Durchführung von Kulturfolgen.

5. In der laut Anspruch 4 eingesetzten Methode sind die zystenbildenden Nematoden eine Solanaceae Kultur.

6. Methode laut Anspruch 4 und 5 in der mindestens eine Anwendung vor der Kultur und/oder während der Kultur und/oder nach jedem Kulturzyklus stattfindet.

## Revendications

1. Le recours au champignon *Paecilomyces carneus,* seul ou combiné à au moins un vecteur, dan sle cadre du contrôle ou de la prévention ou de l'éradication de *Globodera* spp dans des cultures infestées de nématodes à kystes, mis en place en même temps que la rotation des cultures et où le champignon *Paecilomyces carneus* est une masse propagée dans la composition qui comprend de l'avoine, de la levure, du jus de carotte et du chloramphénicol.

2. Au regard de la revendication 1, là où la culture infestée par les nématodes à kystes le recours correspond à une culture de Solanaceae.

3. Au regard des revendications 1 et 2, le recours, seul ou combiné à au moins un vecteur, pour le contrôle ou la prévention ou l'éradication de la *Globodera* spp, là où au moins une application est menée avant la culture et/ou pendant la culture et/ou après chaque cycle de culture.

4. Une méthode pour le contrôle ou la prévention ou l'éradication de la *Globodera* spp dans des cultures infestées par les nématodes à kystes, dite méthode comprenant :
a. à travers l'inoculation de cellules vivantes de *Paecilomyces carneus* dans un milieu liquide comprenant de l'avoine, de la levure, du jus de carotte et du chloramphénicol ;
b. à travers l'incubation de la culture à une température comprise entre 12°C et 35°C pour une durée suffisante afin d'obtenir la concentration désirée ;
c. à travers la mise en place de cellules vivantes de *Paecilomyces carneus* ainsi obtenues, seules ou combinées avec au moins un vecteur à des cultures infestées par des nématodes à kystes, en même temps que la rotation de al culture.

5. Au regard de la revendication 4, là où la culture est infestées par des nématodes à kystes, la méthode correspond à une culture de Solanaceae.

6. Au regard des revendications 4 à 5, la méthode correspond à une application e menée avant la culture et/ou pendant al culture et/ou après chaque cycle de culture.
